Europäisches Patentamt

European Patent Office

Office européen des brevets

(19)

(11) Publication number: **0 011 997**
**B1**

(12)

# EUROPEAN PATENT SPECIFICATION

(45) Date of publication of patent specification: **21.07.82**

(21) Application number: **79302680.8**

(22) Date of filing: **23.11.79**

(51) Int. Cl.³: **B 01 D 53/14** //C01B3/52, C10K1/14

(54) **Method for removing hydrogen sulphide from gaseous mixtures containing hydrogen sulphide and carbon dioxide.**

(30) Priority: **23.11.78 GB 4574778**

(43) Date of publication of application:
**11.06.80 Bulletin 80/12**

(45) Publication of the grant of the patent:
**21.07.82 Bulletin 82/29**

(84) Designated Contracting States:
**BE DE FR IT NL**

(56) References cited:
**DE - A - 1 903 693**
**DE - A - 2 548 700**
**FR - A - 2 240 282**

(73) Proprietor: **AIR PRODUCTS AND CHEMICALS, INC.**
**P.O. Box 538**
**Allentown, Pennsylvania 18105 (US)**

(72) Inventor: **Antonas, Ioannis Antonios**
**102 Ledbury Road**
**London W.11. (GB)**

(74) Representative: **Lucas, Brian Ronald**
**c/o Air Products Limited Hersham Place Molesey Road**
**Walton-on-Thames Surrey, KT12 4RZ (GB)**

Courier Press, Leamington Spa, England.

**0011997**

### Method for removing hydrogen sulphide from gaseous mixtures containing hydrogen sulphide and carbon dioxide

This invention relates to a method for removing hydrogen sulphide from gaseous mixtures comprising hydrogen sulphide and carbon dioxide.

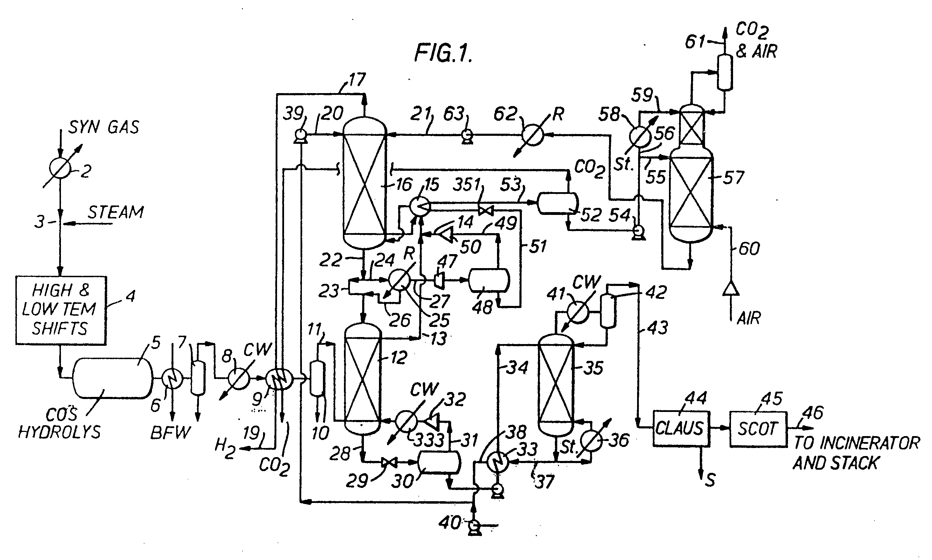

German Patent Specification No. DE—A—2,548,700 disclosed a process for removing carbon dioxide and hydrogen sulphide from a gas stream by washing the gas stream with methanol in an absorption column. As can be seen in Figure 1 of that Specification, the gas stream is introduced into the bottom (first stage) of a three stage absorption column. Substantially all the hydrogen sulphide is absorbed in the first stage together with some carbon dioxide. The bulk of the carbon dioxide is then absorbed in the second and third stages of the absorption column. Liquid methanol is introduced into the third stage of the absorption column where it absorbs carbon dioxide. This is an exothermic process and since the ability of the methanol to absorb carbon dioxide decreases as the temperature rises the methanol is withdrawn from the bottom of the third stage of the column, cooled and returned to the second stage of the column. Thus, whilst the methanol leaves the column substantially saturated with carbon dioxide at the temperature and partial pressure of carbon dioxide at the bottom of the third stage of the column it returns to the top of the second stage in an unsaturated state and can absorb more carbon dioxide.

Usually the amount of hydrogen sulphide to be removed is far smaller than the amount of carbon dioxide and for this reason the methanol leaving the second stage (which methanol is saturated with carbon dioxide at the temperature and partial pressure of the carbon dioxide at the exit of the second stage) is divided into two portions. One portion is regenerated to release carbon dioxide whilst the other portion is passed through the first stage to absorb hydrogen sulphide. Since the partial pressure of carbon dioxide in the first stage is necessarily higher than that in the second stage, some carbon dioxide is unavoidably co-absorbed with the release of heat. As will become apparent from the following description significant advantages can be derived by inhibiting the absorption of carbon dioxide in the region where hydrogen sulphide is absorbed. This is achieved by introducing additional carbon dioxide into the absorber en-route for the hydrogen sulphide absorption section by exposing the absorbent to an atmosphere containing carbon dioxide at a higher partial pressure that was in the $CO_2$ absorption stage. The advantages offered by this step are greatly enhanced by regenerating the absorbent leaving the hydrogen sulphide absorption stage thermally rather than by the usual pressure drop technique described herein with reference to Figure 1. It should however be conceded that thermal regeneration *per se* is shown in the prior art, for example German Patent Specification No. DE—A—1,903,693.

Figure 1 of the accompanying drawings shows a known installation for recovering a hydrogen rich stream from synthesis gas. In particular, 1817.3 Kg moles/hr (4006.4 lb moles/hr) of synthesis gas comprising:

| | |
|---|---|
| 796.7 Kg moles | (1756.5 lb moles) hydrogen |
| 10.1 Kg moles | ( 22.2 lb moles) methane |
| 2.9 Kg moles | ( 6.4 lb moles) water |
| 113.1 Kg moles | ( 249.3 lb moles) carbon dioxide |
| 871.5 Kg moles | (1921.3 lb moles) carbon monoxide |
| 19.0 Kg moles | ( 41.9 lb moles) hydrogen sulphide |
| 3.2 Kg moles | ( 7.0 lb moles) nitrogen |
| 0.8 Kg moles | ( 1.8 lb moles) carbonyl sulphide |

at 45.5 bars A (660 psia) and 50°C (122°F) is heated to 154°C (310°F) in heat exchanger 2. 2524.4 Kg moles/hr (5565.4 lb moles/hr) of steam at 53.1 bars A (770 psia) and 267.8°C (514°F) are added to the synthesis gas at junction 3 and the resulting mixture is subjected to high and low temperature shift reactions in unit 4. The carbonyl sulphide is substantially hydrolysed in unit 5 and the gas leaving unit 5 is cooled to 60°C (140°F) in heat exchanger 6. Substantially all the water which condenses in heat exchanger 6 is separated from the gas in separator 7 and the remaining gas is cooled to 12.8°C (55°F) in heat exchangers 8 and 9. The water which condenses in heat exchangers 8 and 9 is separated from the gas in phase separator 10 and the residual gas at 4319 bars A (637 psia) and comprising (per hour)

| | | | |
|---|---|---|---|
| 1661.1 | Kg moles | (3662.1 lb moles) | hydrogen |
| 10.1 | Kg moles | ( 22.2 lb moles) | methane |
| 0.8 | Kg moles | ( 1.7 lb moles) | water |
| 978.2 | Kg moles | (2156.6 lb moles) | carbon dioxide |
| 7.1 | Kg moles | ( 15.7 lb moles) | carbon monoxide |
| 19.8 | Kg moles | ( 43.7 lb moles) | hydrogen sulphide |
| 3.2 | Kg moles | ( 7.0 lb moles) | nitrogen |
| | | 1.6 ppm | carbonyl sulphide |

enters adsorber 12 through pipe 11.

Substantially all the hydrogen sulphide in the residual gas is absorbed in absorber 12 and the gas leaving adsorber 12 through pipe 13 contains approximately 0.5 ppm hydrogen sulphide. The gas in pipe 13 is joined by gas from conduit 14 comprising (per hour)

| 10.7 | Kg moles | (23.5 | lb moles) | hydrogen |
|---|---|---|---|---|
| 0.1 | Kg moles | ( 0.2 | lb moles) | methane |
| 0.004 | Kg moles | ( 0.01 | lb moles) | water |
| 19.9 | Kg moles | (43.8 | lb moles) | carbon dioxide |
| 0.1 | Kg moles | ( 0.2 | lb moles) | carbon monoxide |
| 0.02 | Kg moles | ( 0.05 | lb moles) | nitrogen |

The resulting gas mixture is cooled to 2°C (34°F) in heat exchanger 15 and is then introduced into the bottom of a second absorber 16 where a substantial portion of the remaining carbon dioxide is removed.

The gas stream leaves the second adsorber 16 through conduit 17 at −3°C (26°F) and passes through heat exchanger 9 which it leaves through conduit 19 at 29°C (84°F). The gas in conduit 19 is at 43.7 bars A (634 psia) and contains (per hour)

| 1656.3 | Kg moles | (3651.6 | lb moles) | hydrogen |
|---|---|---|---|---|
| 9.8 | Kg moles | ( 21.5 | lb moles) | methane |
| 0.1 | Kg moles | ( 0.3 | lb moles) | water |
| 1.0 | Kg moles | ( 2.1 | lb moles) | carbon dioxide |
| 7.1 | Kg moles | ( 15.6 | lb moles) | carbon monoxide |
| | | 0.1 | ppm | hydrogen sulphide |
| 3.1 | Kg moles | ( 6.9 | lb moles) | nitrogen |
| | | 0.3 | ppm | carbonyl sulphide |

The absorbent used in the process described is a mixture of ethers sold under the Trade Name SELEXOL. 90718 Kg/hr (200,000 lbs/hr) SELEXOL together with 206.1 Kg moles (454.3 lb moles) water enter second adsorber 16 through conduit 20 at 44.8 bars A (650 psia) and at −4°C (24°F). Simultaneously, 287576 Kg moles/hr (634,000 lbs/hr) SELEXOL and 652.4 kg moles (1438.3 lb moles) water enter second absorber 16 through conduit 21 at 44 bars A (640 psia) and −4°C (24°F). As the SELEXOL descends through the second absorber 16 it absorbs carbon dioxide and, in so doing, increases in temperature. The absorbent leaves the bottom of the second absorber 16 through conduit 22 at +1°C (34°F). The absorbent is then divided into two streams 23 and 24. Stream 24 is cooled to −4°C (25°F) in refrigerator 25 and part is passed through conduit 26 whilst the remainder is passed through conduit 27. Streams 23 and 26 are combined and the remaining stream at 0°C (32°F) enters absorber 12 at 43.7 bars A (634 psia). As the stream descends through absorber 12 it absorbs hydrogen sulphide and leaves absorber 12 through conduit 28 at 10°C (50°F) and 43.9 bars A (637 psia). The stream is expanded to 1.93 bars A (28 psia) across valve 29 and in so doing cools to −13°C (9°F). The expansion releases a substantial quantity of gas from the stream and this is separated from the liquid in separator 30. The gas leaving separator 30 through conduit 31 comprises (per hour)

| 5.2 | Kg moles | ( 11.5 | lb moles) | hydrogen |
|---|---|---|---|---|
| 0.1 | Kg moles | ( 0.3 | lb moles) | methane |
| 260.1 | Kg moles | (573.5 | lb moles) | carbon dioxide |
| 0.05 | Kg moles | ( 0.1 | lb moles) | carbon monoxide |
| 8.7 | Kg moles | ( 19.3 | lb moles) | hydrogen sulphide |
| | | 0.4 | ppm | carbonyl sulphide |

The gas in conduit 31 is compressed to 44.1 bars A (640 psia) in compressor 32, and after being cooled to 40°C (104°F) in heat exchanger 33 is introduced into absorber 12. The liquid leaving separator 30 comprises (per hour):

| 206.6 | Kg moles | ( 455.6 | lb moles) | water |
|---|---|---|---|---|
| 59.8 | Kg moles | ( 131.9 | lb moles) | carbon dioxide |
| 19.8 | Kg moles | ( 43.7 | lb moles) | hydrogen sulphide |
| | | 0.1 | ppm | carbonyl sulphide |
| 90718 | Kg moles | (200,000 | lbs ) | SELEXOL |

The liquid is heated to 119°C (247°F) in heat exchanger 33 and passed through conduit 34 into desorber 35. Part of the liquid leaving the bottom of the desorber 35 is vapourised in heat exchanger 36 and returned to the desorber 35 as reboil whilst the remainder passes through conduit 37 and heat exchanger 33. The liquid leaving heat exchanger 33 through conduit 38 is at 1.93 bars A (28 psia) and is recycled to pump 39 where it is compressed to 44.8 bars A (650 psia). Make-up SELEXOL and water is introduced into the system by pump 40.

Part of the gas leaving the top of absorber 35 is condensed in heat exchanger 41 and returned to the desorber 35 via separator 42 as reflux. The residual gas comprising

| 4.1 | Kg moles | ( 9.0 | lb moles) | water |
|---|---|---|---|---|
| 59.8 | Kg moles | (131.9 | lb moles) | carbon dioxide |
| 19.8 | Kg moles | ( 43.7 | lb moles) | hydrogen sulphide |

passes along line 43 to Claus unit 44 and SCOT unit 45 where substantially all the hydrogen sulphide is removed. The carbon dioxide, water vapour and any residual hydrogen sulphide are passed through an incinerator and vented to atmosphere via conduit 46.

The residual stream 27 is expanded to 20.7 bars A (300 psia) in expander 47 and the gas and liquid thus formed are separated in separator 48. The gas phase leaves separator 48 through conduit 49 and is compressed to 44.1 bars A (640 psia) in compressor 50. The liquid phase leaves separator 48 through conduit 51 and is expanded to 1.52 bars A (22 psia) across valve 351. The expansion causes the temperature to drop and the two phase mixture formed by the expansion is warmed to −0.5°C (31°F) in heat exchanger 15 before entering phase separator 52 through conduit 53. The gaseous phase leaving separator 52 passes through heat exchanger 9 and leaves the plant at 29°C (84°F) and 1.24 bars A (18 psia). The gaseous phase comprises (per hour)

| 4.8 | Kg moles | ( 10.5 | lb moles) | hydrogen |
|---|---|---|---|---|
| 0.3 | Kg moles | ( 0.7 | lb moles) | methane |
| 813.9 | Kg moles | (1794.4 | lb moles) | carbon dioxide |
| 1.9 | Kg moles | ( 4.1 | lb moles) | water |
| 0.05 | Kg moles | ( 0.1 | lb moles) | carbon monoxide |
| 0.05 | Kg moles | ( 0.1 | lb moles) | nitrogen |
| | | 0.8 | ppm | carbonyl sulphide |

The liquid leaving separator 52 passes through pump 54 and is divided into two streams 55 and 56. Stream 55 is introduced directly into desorber 57 whilst stream 56 is heated in heat exchanger 58 before entering desorber 57 through conduit 59.

Air at 27°C (80°F) and 1.4 bars A (20 psia) is introduced into the bottom of desorber 57 through conduit 60 and substantially all the carbon dioxide is desorbed and leaves the desorber 57 through vent 61 together with the air. The liquid leaving the bottom of desorber 57 comprises (per hour)

| 652.4 | Kg moles | (1438.3 | lb moles) | water |
|---|---|---|---|---|
| 287576.0 | Kg moles | (634,000 | lbs ) | SELEXOL |

and is at 1.4 bars A (20 psia) and −0.5°C (31°F). The liquid is cooled to −4°C (24°F) in refrigerator 62 and is pumped to 44.1 bars A (640 psia) in pump 63 before being recycled through second absorber 16.

Excluding the power requirements for the high and low temperature shift unit 4 and the energy requirements of the apparatus described can be summarized as

| 4.52 | tonnes | (4.45 tons/hr steam (1 ton=2240 lbs)) |
|---|---|---|
| 2424.5 | KW | (3250 hp) |

Of the power requirements 1112.3 KW (1491 hp) is required to drive compressor 32. Furthermore, the compressor 32 represents a major item of capital expenditure, i.e. about 11.6% of the cost of the plant downstream of phase separator 7.

It is an object of at least preferred embodiments of the present invention to provide a plant which will have a lower capital cost and a lower power consumption than that described with reference to Figure 1.

It should perhaps be emphasized that the process described with reference to Figure 1 represents a refined state of a process which has been commercially used for about 20 years.

The present invention is based on the concept of, in combination:

i) introducing additional $CO_2$ into the absorbent entering absorber 12 and

ii) replacing valve 29 with a heat exchanger to heat the liquid leaving absorber 12 through conduit 28.

Referring to item i) it should be understood that whilst the absorbent leaving absorber 16 is saturated with respect to the partial pressure of carbon dioxide present at the bottom of absorber 16 it will still absorb carbon dioxide if it is brought into contact with a gas containing carbon dioxide at a greater partial pressure.

4

Referring to item ii), by applying sufficient heat to the liquid sufficient phase separation can occur in separator 30 and compressor 32 can be dispensed with.

Our calculations indicate that there is both an energy and a capital saving to be made by modifying the design in this manner.

According to the present invention there is provided a method of removing hydrogen sulphide and carbon dioxide from a gaseous mixture comprising hydrogen sulphide and carbon dioxide, which method comprises the steps of:

i) passing said gaseous mixture through a first absorption stage to absorb hydrogen sulphide from said gaseous mixture;

ii) passing the remaining gaseous mixture leaving said first absorption stage through a second absorption stage to absorb carbon dioxide from said remaining gaseous mixture;

iii) introducing a liquid absorbent into said second absorption stage to absorb said carbon dioxide;

iv) dividing the liquid absorbent containing carbon dioxide from said second absorption stage into a first stream and a second stream;

v) desorbing carbon dioxide from said first stream;

vi) cooling said second stream;

vii) introducing said second stream into said first absorption stage;

viii) removing the liquid absorbent containing hydrogen sulphide and absorbed carbon dioxide from said first absorption stage;

ix) treating said removed liquid absorbent to desorb at least a portion of the coabsorbed carbon dioxide therein and recycling at least part of said desorbed carbon dioxide to said first absorption stage, and subsequently

x) depressurizing said absorbent and desorbing the hydrogen sulphide from said liquid absorbent, characterized in that additional $CO_2$ is introduced into said second stream by bringing said second stream into contact with a gas stream rich in carbon dioxide and further characterized in that step (ix) is carried out by heating said liquid absorbent.

Preferably, the temperature of the absorbent in said first absorber is maintained between −18°C (0°F) and 10°C (50°F) and more preferably between −18°C (0°F) and 1°C (34°F).

Preferably, the desorbed hydrogen sulphide is passed through one or more Claus reactors.

Conveniently, carbon dioxide is introduced into said second stream by bringing said second stream into contact with a gas stream rich in carbon dioxide derived from the desorption of carbon dioxide from the liquid absorbent leaving the second absorption stage.

Whilst the present invention is particularly suited to the purification of synthesis gas (which may or may not be shifted) it is also suitable for the purification of other gaseous mixtures, for example natural gas and synthetic natural gas.

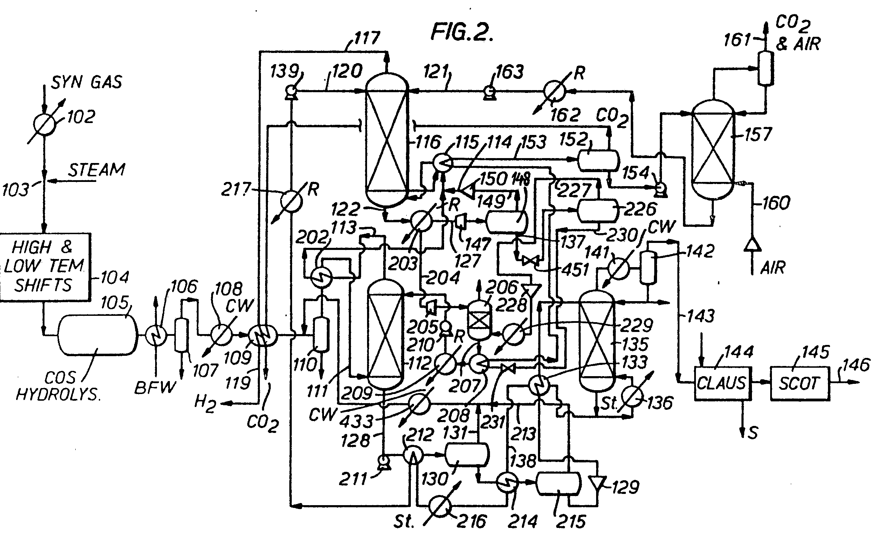

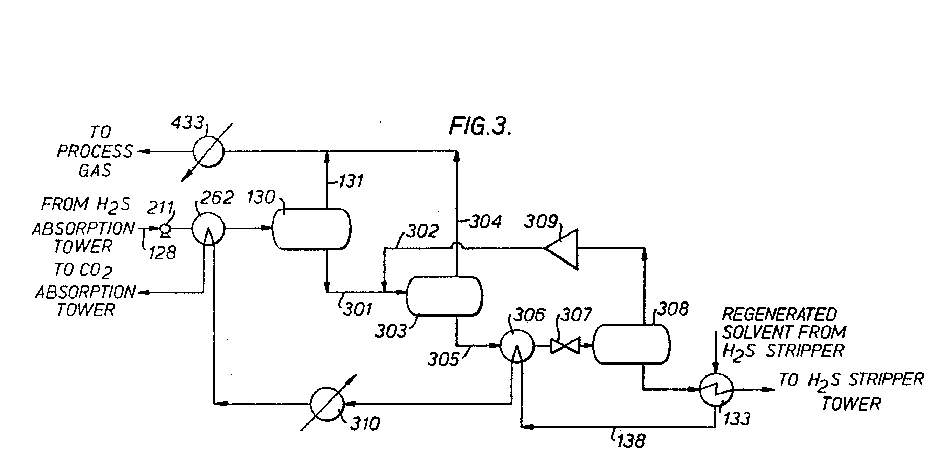

For a better understanding of the present invention reference will now be made, by way of example, to Figure 2 of the accompanying drawings which is a simplified flowsheet of a plant in accordance with the present invention and to Figure 3 which shows a modification to the plant shown in Figure 2.

Referring to Figure 2, 1817.3 Kg moles/hr (4006.4 lb moles/hr) of synthesis gas comprising:

| | | | | |
|---|---|---|---|---|
| 796.7 | Kg moles | (1756.5 | lb moles) | hydrogen |
| 10.1 | Kg moles | ( 22.2 | lb moles) | methane |
| 2.9 | Kg moles | ( 6.4 | lb moles) | water |
| 113.1 | Kg moles | ( 249.3 | lb moles) | carbon dioxide |
| 871.5 | Kg moles | (1921.3 | lb moles) | carbon monoxide |
| 19.0 | Kg moles | ( 41.9 | lb moles) | hydrogen sulphide |
| 3.2 | Kg moles | ( 7.0 | lb moles) | nitrogen |
| 0.8 | Kg moles | ( 1.8 | lb moles) | carbonyl sulphide |

at 45.5 bars A (660 psia) and 50°C (122°F) is heated to 154°C (310°F) in heat exchanger 102. 2524.4 Kg moles/hr (5565.4 lb moles/hr) of steam at 53.1 bars A (770 psia) and 268°C (514°F) are added to the synthesis gas at junction 103. The resulting mixture is subjected to high and low temperature shift reactions in unit 104 and the carbonyl sulphide is substantially hydrolysed in unit 105. The gas leaving unit 105 is cooled to 60°C (140°F) in heat exchanger 106 and substantially all the water which condenses in heat exchanger 106 is separated from the gas in separator 107. The remaining gas is cooled to 13°C (55°F) in heat exchangers 108 and 109. The water which condenses in heat exchangers 108 and 109 is separated from the residual gas in phase separator 110 and the residual gas at 43.9 bars A (637 psia) and 13°C (55°F) and comprising (per hour):

| | | | | |
|---|---|---|---|---|
| 1660.4 | Kg moles | (3660.6 | lb moles) | hydrogen |
| 10.1 | Kg moles | ( 22.2 | lb moles) | methane |
| 12.2 | Kg moles | ( 26.8 | lb moles) | water |
| 978.2 | Kg moles | (2156.6 | lb moles) | carbon dioxide |
| 7.1 | Kg moles | ( 15.7 | lb moles) | carbon monoxide |

5

| | | | | |
|---|---|---|---|---|
| 19.8 | Kg moles | ( 43.7 | lb moles) | hydrogen sulphide |
| 3.2 | Kg moles | ( 7.0 | lb moles) | nitrogen |
| | | 1.4 | ppm | carbonyl sulphide |

is cooled to 1°C (34°F) in heat exchanger 202 before being introduced into absorber 112 through pipe 111.

Substantially all the hydrogen sulphide in the residual gas is removed in absorber 112 and the gas leaving absorber 112 at −16°C (4°F) through pipe 113 contains approximately 0.5 ppm hydrogen sulphide. The gas is heated in heat exchanger 202 and is then joined by gas from conduit 114 comprising (per hour):

| | | | | |
|---|---|---|---|---|
| 12.0 | Kg moles | (26.5 | lb moles) | hydrogen |
| 0.1 | Kg moles | ( 0.2 | lb moles) | methane |
| 12.5 | Kg moles | (27.5 | lb moles) | carbon dioxide |
| 0.1 | Kg moles | ( 0.3 | lb moles) | carbon monoxide |
| 0.03 | Kg moles | ( 0.07 | lb moles) | nitrogen |
| | | 0.2 | ppm | carbonyl sulphide |

The water in the resulting gas mixture is partially condensed in heat exchanger 115 and is then introduced into the bottom of a second absorber 116 where a substantial portion of the remaining carbon dioxide is removed.

The gas stream leaves the second absorber 116 through conduit 117 at 3°C (26°F) and passes through heat exchanger 109 which it leaves through conduit 119 at 29°C (84°F).

The absorbent used in the process described with reference to Figure 2 is again SELEXOL. 45,359 Kg/hr (100,000 lbs/hr) SELEXOL together with 27 lb moles (60 lb moles) water enter second absorber 116 through conduit 120 at 44.1 bars A (640 psia) and 4°C (24°F). Simultaneously, 332935 Kg/hr (734,000 lbs/hr) SELEXOL and 324.3 Kg moles (714.9 lb moles) of water enter second absorber 116 through conduit 121 at 44.1 bars A (640 psia) and 4°C (24°F). As the SELEXOL descends through the second absorber 116 it absorbs carbon dioxide and, in so doing, increases in temperature. The absorbent leaves the bottom of the second absorber 116 through conduit 122 at 1°C (34°F) and is then cooled to 4°C (25°F) in refrigerator 203. The cooled liquid is divided into two streams 204 and 127. Stream 204 is expanded to 420 psia in expander 205. The liquid leaving expander 205 is introduced into a column 206 where it is saturated with carbon dioxide. The saturated liquid leaves column 206 through pipe 207 and, after being cooled to −15°C (5°F) in heat exchanger 208 and −18°C (0°F) in refrigerator 209 is pumped to 635 psia by pump 210 before entering absorber 112. As the liquid descends through absorber 112 it absorbs hydrogen sulphide and in so doing is warmed to 1°C (34°F) as it leaves absorber 112 through conduit 128. The liquid is pumped to 44.1 bars A (640 psia) by pump 211 and is then heated to 71°C (160°F) in heat exchanger 212. The resulting two phase mixture is separated in separator 130. The gas leaving separator 130 through conduit 131 comprises (per hour):

| | | | | |
|---|---|---|---|---|
| 2.0 | Kg moles | ( 4.3 | lb moles) | hydrogen |
| 0.1 | Kg moles | ( 0.2 | lb moles) | methane |
| 0.2 | Kg moles | ( 0.4 | lb moles) | water |
| 80.7 | Kg moles | (178 | lb moles) | carbon dioxide |
| 3.5 | Kg moles | ( 7.8 | lb moles) | hydrogen sulphide |
| | | 0.3 | ppm | carbonyl sulphide |

The gas in conduit 131 is joined by gas from conduit 213 and the combined stream is cooled to 35°C (95°F) in heat exchanger 433 before being introduced into the feed stream immediately upstream of separator 110. The liquid leaving separator 130 is heated to 131°C (268°F) in heat exchanger 214 and the resultant two phase mixture is separated in separator 215. The gas phase leaving separator 215 passes through conduit 213 whilst the liquid phase is expanded through expander 129 to 1.93 bars A (28 psia). The expanded liquid is heated in heat exchanger 133 and is passed through conduit 134 into desorber 135. Part of the liquid leaving the bottom of the desorber 135 is vaporized in heat exchanger 136 and returned to the desorber as reboil whilst the remainder passes through heat exchanger 133. The liquid leaving the heat exchanger 133 through conduit 138 is at 1.8 bars A (26 psia) and 150°C (302°F). Heat is extracted from this liquid in heat exchanger 214 and the liquid is then heated to 90°C (195°F) in heat exchanger 216. The hot liquid is passed through heat exchanger 212 which it leaves at 7°C (44°F). The liquid is then cooled to −4°C (24°F) in refrigerator 217 before being pumped to 44.1 bars A (640 psia) by pump 139.

Part of the water in the gas leaving the top of absorber 135 is condensed in heat exchanger 141 and returned to desorber 135 via separator 142 as reflux. The residual gas comprising (per hour):

| | | | | |
|---|---|---|---|---|
| 3.4 | Kg moles | ( 7.4 | lb moles) | water |
| 43.5 | Kg moles | (96 | lb moles) | carbon dioxide |
| 19.8 | Kg moles | (43.7 | lb moles) | hydrogen sulphide |

6

is passed along line 143 to Claus unit 144. The tail gases leaving the Claus unit 144 are passed through SCOT unit 145 where substantially all the hydrogen sulphide is removed. The carbon dioxide, water vapour and any residual hydrogen sulphide are passed through an incinerator and vented to atmosphere via conduit 146.

The residual stream 127 is expanded to 20.7 bars A (300 psia) in expander 147 and the gas and liquid thus found are separated in separator 148. The gas phase leaves the separator 148 through conduit 149 and is compressed to 43.7 bars A (634 psia) in compressor 150. The liquid phase leaves separator 148 through conduit 151 and is expanded to 10 bars a (146 psia) across valve 451. The expansion causes the temperature to drop to −6°C (22°F) and the two phase mixture formed by the expansion is passed to separator 226. The gas phase leaving separator 226 comprises almost entirely carbon dioxide with small traces of hydrogen, carbon monoxide and nitrogen. The gas is passed through conduit 227 to compressor 228 where its pressure is raised to 29.4 bars A (426 psia). The gas is then cooled in heat exchanger 229 before being introduced into column 206 where it saturates the SELEXOL with carbon dioxide. The liquid leaving separator 226 through conduit 230 is expanded to 1.79 bars A (26 psia) across valve 231 and is then warmed to −2°C (28°F) in heat exchangers 208 and 115. The two phase mixture leaving heat exchanger 208 through conduit 153 is separated in separator 152. The gaseous phase leaving separator 152 passes through heat exchanger 109 and leaves the plant at 29.0°C (84°F) and 1.52 bars A (22 psia).

The liquid leaving separator 152 passes through pump 154 and is fed directly into desorber 157. Air is introduced into the bottom of desorber through conduit 160 and substantially all the carbon dioxide is desorbed and leaves the desorber 157 through vent 161 together with the air. The liquid leaving the bottom of desorber 157 comprises (per hour):

|  |  |  |  |  |
|---|---|---|---|---|
| 324.3 | Kg moles | ( 714.9 | lb moles) | water |
| 332935 | Kg moles | (734,000 | lbs ) | SELEXOL |

and is at 1.52 bars A (22 psia) and −3°C (26°F). The liquid is cooled to −4°C (24°F) in refrigerator 162 and is pumped to 44.1 bars A (640 psia) in pump 163 before before being recycled through second absorber 116.

Excluding the power requirements for the high and low temperature shift unit 104 and the carbonyl sulphide hydrolysis unit 105 the energy requirements of the apparatus described can be summarized as

|  |  |  |
|---|---|---|
| 3007.5 | Kg moles | (2.96 tons/hr steam (1 ton=2240 lbs)) |
| 1633.7 | Kg moles | (2190 hp) |

It will be seen that the energy consumption of Figure 2 is 33.48% lower than the prior art described with reference to Figure 1 and the horse power requirements are reduced by 32.6%. Furthermore, our calculations indicate that the installed capital cost of the installation shown in Figure 2 should be 18.67% lower than the prior art described with reference to Figure 1.

Various modifications to the installation shown in Figure 2 are currently contemplated. Thus, if the feed mixture has a very low hydrogen sulphide content it may be advantageous to modify the treatment of the liquid leaving the bottom of the absorber 112 as shown in Figure 3. Parts in Figure 3 which have similar counter-parts in Figure 2 are identified by the same reference numeral as used in Figure 2. In particular, the liquid which leaves the bottom of absorber 112 through conduit 128 is pumped through heat exchanger 212 where it is heated to 71°C (160°F). The resulting two phase mixture is separated in separator 130 and the gas phase returned to the absorber feed via cooler 433. The liquid phase leaves separator 130 through conduit 301 and is mixed with gas from conduit 302. The resulting mixture is flashed in vessel 303 and the gaseous phase returned to the absorber feed via conduit 304 and cooler 433. The liquid phase leaves separator 303 through conduit 305 and is heated to 131°C (268°F) in heat exchanger 306 before being flashed to 30 bars A (304 psia) across valve 307. The two phase mixture leaving valve 307 is separated in separator 308 and the gaseous phase is compressed in compressor 309 and passed through conduit 302. The liquid leaving separator 308 is passed through heat exchanger 133 and fed to desorber 135. The liquid leaving the bottom of the desorber is passed through heat exchanger 133 and is then conducted to heat exchanger 306 via conduit 138. The liquid leaving heat exchanger 306 is heated in heat exchanger 310 and is then passed through heat exchanger 212. The liquid leaving the heat exchanger 212 is then cooled in heat exchanger 217 and pumped to the top of the second absorber 116 by pump 139.

The modification shown in Figure 3 requires more power than the installation shown in Figure 2 and is somewhat more expensive. It is still, however, considerably cheaper and more efficient that the arrangement shown in Figure 1. It is believed that the modification will be necessary only in unusual circumstances where the level of hydrogen sulphide in the feed streams is particularly low.

**Claims**

1. A method of removing hydrogen sulphide and carbon dioxide from a gaseous mixture comprising hydrogen sulphide and carbon dioxide, which method comprises the steps of:

# 0011997

i) passing said gaseous mixture through a first absorption stage to absorb hydrogen sulphide from said gaseous mixture;

ii) passing the remaining gaseous mixture leaving said first absorption stage through a second absorption stage to absorb carbon dioxide from said remaining gaseous mixture;

iii) introducing a liquid absorbent into said second absorption stage to absorb said carbon dioxide;

iv) dividing the liquid absorbent containing carbon dioxide from said second absorption stage into a first stream and a second stream;

v) desorbing carbon dioxide from said first stream;

vi) cooling said second stream;

vii) introducing said second stream into said first absorption stage;

viii) removing the liquid absorbent containing hydrogen sulphide and absorbed carbon dioxide from said first absorption stage;

ix) treating said removed liquid absorbent to desorb at least a portion of the coabsorbed carbon dioxide therein and recycling at least part of said desorbed carbon dioxide to said first absorption stage, and subsequently

x) depressurizing said absorbent and desorbing the hydrogen sulphide from said liquid absorbent, characterized in that additional $CO_2$ is introduced into said second stream by bringing said second stream into contact with a gas stream rich in carbon dioxide and further characterized in that step (ix) is carried out by heating said liquid absorbent.

2. A method according to Claim 1, characterized in that the temperature of the liquid absorbent in said first absorption stage is maintained between −18°C (0°F) and 10°C (50°F).

3. A method according to Claim 1 or 2, characterized in that the temperature of the liquid absorbent is maintained at between −18°C (0°F) and 1°C (34°F).

4. A method according to any preceding claim, characterized in that the desorbed hydrogen sulphide is passed through one or more Claus reactors.

5. A method according to any preceding claim, wherein carbon dioxide is introduced into said second stream by bringing said second stream into contact with a gas stream rich in carbon dioxide derived from the desorbtion of carbon dioxide from the liquid absorbent leaving said second absorption stage.

## Revendications

1. Procédé pour éliminer l'acide sulfhydrique et le dioxyde de carbone d'un mélange gazeux comprenant de l'acide sulfhydrique et du dioxyde de carbone, ce procédé comprenant des stades qui consistent à:

1) faire passer ledit mélange gazeux à travers un premier étage d'absorption pour absorber l'acide sulfhydrique dudit mélange gazeux;

2) faire passer le mélange gazeux restant quittant ledit premier étage d'absorption à travers un second étage d'absorption pour absorber le dioxyde de carbone dudit mélange gazeux restant;

3) introduire un absorbant liquide dans ledit second étage d'absorption pour absorber ledit dioxyde de carbone;

4) diviser l'absorbant liquide contenant le dioxyde de carbone dudit second étage d'absorption en un premier courant et un second courant;

5) désorber le dioxyde de carbone dudit premier courant;

6) refroidir ledit second courant;

7) introduire ledit second courant dans ledit premier étage d'absorption;

8) éliminer l'absorbant liquide contenant l'acide sulfhydrique et le dioxyde de carbone absorbé dudit premier étage d'absorption;

9) traiter ledit absorbant liquide éliminé pour en désorber au moins une portion du dioxyde de carbone coabsorbé et recycler au moins une partie dudit dioxyde de carbone désorbé dans ledite premier étage d'absorption, puis,

10) détendre ledit absorbant et désorber l'acide sulhydrique dudit absorbant liquide, caractérisé en ce qu'on introduit du dioxyde de carbone additionnel dans ledit second courant par mise en contact dudit second courant avec un courant gazeux riche en dioxyde de carbone et caractérisé de plus en ce qu'on effectue le stade 9) par chauffage dudit absorbant liquide.

2. Procédé selon la revendication 1, caractérisé en ce que la température de l'absorbant liquide dans ledit premier étage d'absorption est maintenue entre −18°C et 10°C.

3. Procédé selon l'une des revendications 1 ou 2, caractérisé en ce que la température de l'absorbant liquide est maintenue entre −18°C et 1°C.

4. Procédé selon l'une quelconque des revendications précédentes, caractérisé en ce que l'acide sulfhydrique désorbé est conduit à travers un ou plusieurs réacteurs Claus.

5. Procédé selon l'une quelconque des revendications précédentes, dans lequel du dioxyde de carbone est introduit dans ledit second courant par mise en contact dudit second courant avec un courant de gaz riche en dioxyde de carbone dérivant de la désorption du dioxyde de carbone de l'absorbant liquide quittant ledit second étage d'absorption.

8

**0011997**

**Patentansprüche**

1. Verfahren zum Entfernen von Schwefelwasserstoff und Kohlendioxid aus einer gasförmigen Mischung, die Schwefelwasserstoff und Kohlendioxid enthält, wobei das Verfahren folgende Stufen umfaßt:

i) Leiten der gasförmigen Mischung über eine erste Absorptionsstufe, um den Schwefelwasserstoff aus der gasförmigen Mischung zu absorbieren;

ii) Leiten der restlichen gasförmigen Mischung, die in der ersten Absorptionsstufe verbleibt, über eine zweite Absorptionsstufe, um das Kohlendioxid aus der verbleibenden Gasmischung zu entfernen;

iii) Einleiten eines flüssigen Absorptionsmittels in diese zweite Absorptionsstufe, um das Kohlendioxid zu absorbieren;

iv) Aufteilen des flüssigen Absorptionsmittels, das das Kohlendioxid aus der zweiten Absorptionsstufe enthält, in einen ersten und einen zweiten Strom;

v) Desorbieren des Kohlendioxids aus dem ersten Strom;

vi) Abkühlen des zweiten Stroms;

vii) Einleiten des zweiten Stroms in die erste Absorptionsstufe;

viii) Entfernen des flüssigen Absorptionsmittels, das den Schwefelwasserstoff und das Kohlendioxid enthält, das von der ersten Absorptionsstufe absorbiert wurde;

ix) Behandeln des entfernten flüssigen Absorptionsmittels, um mindestens ein Teil des darin mitabsorbierten Kohlendioxids zu desorbieren und Rückführung mindestens eines Teils dieses desorbierten Kohlendioxids zu der ersten Absorptionsstufe, und anschließend

x) Entspannen des Absorptionsmittels und Desorbieren des Schwefelwasserstoffs aus dem flüssigen Absorptionsmittel, dadurch gekennzeichnet, daß man zusätzliches Kohlendioxid in den zweiten Strom einleitet, indem man den zweiten Strom mit einem an Kohlendioxid reichen Gasstrom in Berührung bringt und daß man weiterhin die Stufe (ix) unter Erhitzen des flüssigen Absorptionsmittels durchführt.

2. Verfahren nach Anspruch 1, dadurch gekennzeichnet, daß man die Temperatur des flüssigen Absorptionsmittels in der ersten Absorptionsstufe zwischen −18°C und 10°C hält.

3. Verfahren nach Anspruch 1 oder 2, dadurch gekennseichnet, daß man die Temperatur des flüssigen Absorptionsmittels zwischen −18°C und 1°C hält.

4. Verfahren nach einem der Ansprüche 1 bis 3, dadurch gekennzeichnet, daß man den desorbierten Schwefelwasserstoff durch einen oder mehrere Claus-Reaktoren leitet.

5. Verfahren nach einem der Ansprüche 1 bis 4, dadurch gekennzeichnet, daß man Kohlendioxid in den zweiten Strom einleitet, indem man den zweiten Strom in Berührung mit einem Kohlendioxid reichen Gasstrom bringt, das aus der Desorption des Kohlendioxid im flüssigen Absorptionsmittel stammt, das in der zweiten Absorptionsstufe verbleibt.

FIG.1.

0011997

FIG.2.

FIG.3.